# EUROPÄISCHE PATENTSCHRIFT

(11) **EP 0 433 636 B1**
(45) Veröffentlichungstag und Bekanntmachung des Hinweises auf die Patenterteilung: **05.04.1995**
(21) Anmeldenummer: 90121270.4
(22) Anmeldetag: 07.11.1990
(51) Int. Cl.: A61F 2/52, B29C 65/50, B29D 22/00, B29C 70/00

(54) **Verfahren zur Herstellung von Brustprothesen**
Process for the production of breast prosthesis
Procédé pour la fabrication d'une prothèse de sein

(30) Priorität: 22.12.1989 DE 3942608
(43) Veröffentlichungstag der Anmeldung: 26.06.1991
(73) Patentinhaber: Thämert Orthopädische Hilfsmittel GmbH & Co., 30938 Burgwedel (DE)
(72) Erfinder: Blau, Hans Georg, W-3006 Burgwedel 1 (DE)
(74) Vertreter: Wehser, Wulf, Dipl.-Ing.

(56) Entgegenhaltungen:
- EP-A- 0 116 357
- EP-A- 0 125 400
- EP-A- 0 320 590
- DE-A- 2 701 627
- DE-A- 3 416 240
- FR-A- 2 405 123
- GB-A- 2 021 478

## Beschreibung

Die Erfindung betrifft ein Verfahren zur Herstellung von Brustprothesen.

Zur Herstellung von Brustprothesen ist es bekannt, in einen Beutel aus zwei an ihren Rändern miteinander verschweißten Polyurethanfolien Silikon einzufüllen. Ein solcher Verfahren geht beispeilsweise aus der EP-A-0.320.590 hervor. Um die Schweißnaht anbringen zu können, ist es daher erforderlich, daß die beiden Polyurethanfolien, also die Bodenfolie und die Deckfolie im Schweißbereich genau fixiert aufeinander liegen, was vergleichsweise arbeitsintensiv und aufwendig ist.

Der Erfindung liegt demgemäß die Aufgabe zugrunde, ein Verfahren und eine Brustprothese zu schaffen, bei welcher die Boden- und Deckfolie auf einfachere Weise aber mit dem gleichen Ergebnis miteinander verbunden werden.

Diese Aufgabe wird erfindungsgemäß dadurch gelöst, daß zwischen die beiden Polyurethanfolien eine Schmelzklebefolie eingelegt wird, die mit einer Ausnehmung versehen ist und die Form eines Ringes hat, wobei die drei so übereinander angeordneten Folien auf den Rand eines Hohlkörperwerkzeuges aufgelegt werden, worauf das Werkzeug unter Offenlassen eines Zuführkanales, vorzugsweise in Form eines Zuführrohres geschlossen wird und anschließend die Füllung erfolgt, indem das Silikonmaterial über den Zuführkanal zwischen die beiden äußeren Folien eingeführt wird, worauf während der Temperaturbehandlung des Werkzeuges für den Vernetzungsvorgang des Silikons die Verbindung zwischen den drei Folien erfolgt.

Zweckmäßigerweise besteht die Schmelzklebefolie ebenfalls aus Polyurethan.

Die Boden- und Deckfolien haben normalerweise eine Stärke bis 0,08 mm, während die Schmelzklebefolie etwa 0,03 mm stark sein kann.

Das erfindungsgemäße Verfahren läßt sich insbesondere auch zur Herstellung von Brustprothesen anwenden, bei denen übereinander mehrere Kammern angeordnet sind. In diesem Fall wird wenigstens eine Schmelzklebefolie verwendet, die durchgehend ohne Ausnehmung ausgebildet ist. Durch zwei voneinander getrennte Zuführkanäle können dann die beiden zwischen der Schmelzklebefolie und der Bodenfolie einerseits und der Schmelzklebefolie und der Deckfolie andererseits gebildeten Kammern getrennt gefüllt werden, wobei insbesondere die Möglichkeit besteht, Füllungen unterschiedlicher Härte, Dichte und/oder Konsistenz zu verwenden.

In einfacher Weise schafft das erfindungsgemäße Verfahren und die erfindungsgemäße Anordnung die Möglichkeit, auch drei und mehr Kammern übereinander anzuordnen, falls dies für Spezialzwecke erforderlich ist. Auch in diesem Falle werden mehrere Klebefolien übereinander im Werkzeug angeordnet, wobei die dritte und die folgenden Kammern jeweils zwischen zwei benachbarten Klebefolien gebildet werden.

Schließlich ist es mit dem erfindungsgemäßen Verfahren möglich, Prothesenmaterial im nicht verflüssigten Zustand, also als fertiges Bauteil, in eine Brustprothese einzubringen. Dies ist insbesondere für die Herstellung von Leichtprothesen von Vorteil.

In diesem Fall wird das zusätzliche Bauteil auf die entweder durchgehend oder ringförmig ausgebildete Schmelzklebefolie aufgebracht, beispielsweise ein Schaumstoffkörper, wobei es möglich ist, diesen Körper auf der Schmelzklebefolie entstehen zu lassen, beispielsweise durch Aufschäumen. Entsprechendes gilt für weiteres Material, wie Watte oder dergleichen, welches teilweise den silikongefüllten Körper ersetzen soll.

Bei diesem Verfahren kann ein silikongefüllter schalenförmiger Prothesenkörper verwendet werden, der aus zwei Polyurethanfolien besteht und eine Höhlung aufweist, die insbesondere dem Körper der Trägerin zugewandt ist. Diese Höhlung kann dann durch einen Schaumstoffkörper oder dergleichen oder auch durch Wette oder mit einer Kombination solcher Materialien ausgefüllt, ausgesteift usw. werden, wobei die Verbindung mit dem schalenförmigen Prothesenkörper durch eine Schmelzklebefolie herbeigeführt wird, welche den Zusatzkörper trägt. Durch Erhitzen der zusammengestellten Prothese in einem entsprechenden Werkzeug wird die Verbindung zu dem Zusatzkörper hergestellt.

Die Erfindung wird im folgenden anhand von Ausführungsbeispielen in der Zeichnung näher erläutert.
- Fig. 1: zeigt in perspektivischer Darstellung eine Brustprothese, auf welche die Erfindung Anwendung findet.
- Fig. 2: ist der Schnitt II-II durch die Brustprothese nach Fig. 1 im umgekehrten Ausgangszustand, in welchem die Folien in ein Werkzeug eingelegt aber noch nicht miteinander verbunden sind.
- Fig. 3: ist die Ansicht III-III nach Fig. 2.
- Fig. 4: ist ein Schnitt ähnlich Fig. 2 bei einer anderen Ausführungsform.
- Fig. 5: ist ein Schnitt ähnlich Fig. 2 bei einer weiteren Ausführungsform.
- Fig. 6: zeigt in Draufsicht eine als Leichtprothese ausgestaltete Brustprothese.
- Fig. 7: ist der Schnitt VII-VII nach Fig. 6.
- Fig. 8: ist ein Schnitt ähnlich Fig. 7 bei einer abgewandelten Ausführungform der Brustprothese.

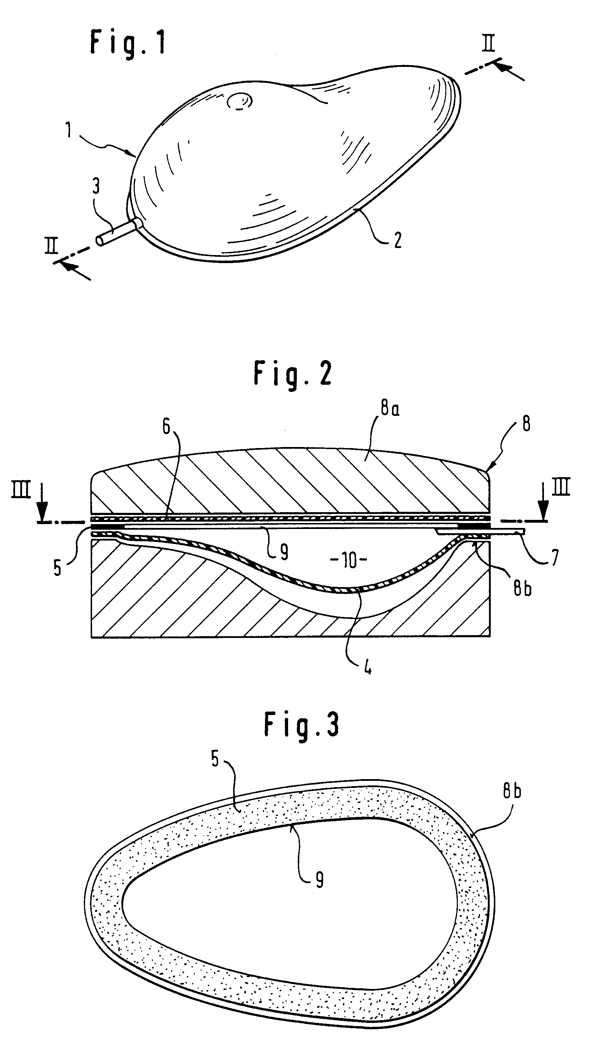

Gemäß Fig. 1 besteht eine Brustprothese 1, auf welche die Erfindung Anwendung findet, aus zwei Polyurethanfolien, die längs eines Randes 2 miteinander verbunden sind. Der so gebildete Beutel wird über ein Zuführrohr 3 mit additionsvernetzendem Silikon gefüllt, wobei die Brustprothese nach der Füllung zur Vernetzung einer Wärmebehandlung unterzogen wird.

Fig. 2 ist der Schnitt II-II bei umgekehrter Brustprothese, die nach dem erfindungsgemäßen Verfahren hergestellt wird. Die erfindungsgemäß hergestellte Brustprothese ist in Fig. 2 im Ausgangszustand wiedergegeben.

Sie besteht aus einer Bodenfolie 4, einer Schmelzklebefolie 5 und einer Deckfolie 6, die gemeinsam unter Einschluß eines Zuführrohres 7 in ein Hohlkörperwerkzeug 8 eingelegt werden.

Die Schmelzklebefolie 5 hat bei der Ausführungsform nach Fig. 2 die Form eines Ringes mit einer mittleren Ausnehmung 9, so daß über das Zufuhrrohr 7 die gesamte von der Bodenfolie 4 und der Deckenfolie 6 umschlossene einzige Kammer 10 mit Silikon gefüllt werden kann.

Nach Einlegen der Folien in der beschriebenen und dargestellten Weise wird der Deckel 8a des Werkzeuges geschlossen, so daß die drei Folien gegeneinander gedrückt werden. Anschließend wird innerhalb des Werkzeuges die Form mit Silikon gefüllt, so daß sich die Folien an die Wandungen anlegen. Im geschlossenen Zustand der Form erfolgt die Wärmebehandlung des Silikons, die gleichzeitig dazu führt, daß die Schmelzklebefolie 5 dem Klebebereich zugeführt wird, so daß sie mit den beiden benachbarten Folien 4 und 6 eine innige Verbindung eingeht.

Fig. 3 ist die Ansicht III-III nach Fig. 2 und zeigt die Schmelzklebefolie 5 in ringförmiger Ausbildung, indem sie die Ausnehmung 9 umschließt. Zur Verdeutlichung ist der Rand des Werkzeuges 8 in Fig. 3 angedeutet, tatsächlich ist es aber zweckmäßig, wenn die Folien voll auf dem Rand 8b (vgl. Fig. 2) aufliegen und diesen eher überragen, so daß er in der Ansicht gemäß Fig. 3 nicht zu sehen ist.

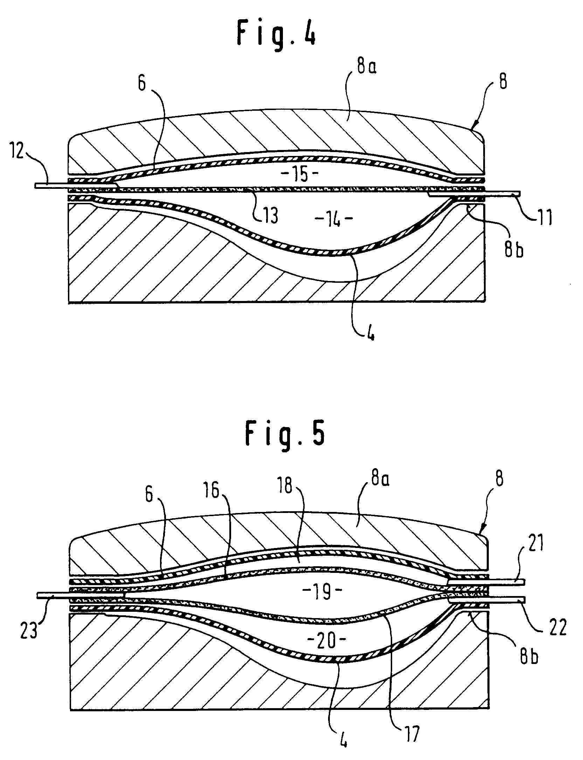

Fig. 4 zeigt eine Ausführungsform, bei welcher ebenfalls eine Klebefolie Verwendung findet, die hier mit 13 bezeichnet ist und die keine Ausnehmung aufweist. Die Schmelzklebefolie 13 teilt mithin den aus den Folien 4 und 6 gebildeten Beutel in zwei Kammern 14 und 15 auf, die über getrennte Zuführrohre 11 und 12 mit Silikon gefüllt werden können. Die Verwendung unterschiedlicher Werkstoffe, insbesondere von Werkstoffen unterschiedlicher Elastizität oder Konsistenz ist möglich.

Bei der Ausführungsform nach Fig. 5 werden zwei durchgehende Schmelzklebefolien 16 und 17 verwendet, die zwischen den Polyurethanfolien 4 und 6 angeordnet sind, so daß drei Kammern 18, 19 und 20 entstehen, die ebenfalls mit unterschiedlichen Werkstoffen gefüllt werden können. Den Kammern 18, 19 und 20 ist je ein Zuführrohr 21, 22 und 23 zugeordnet.

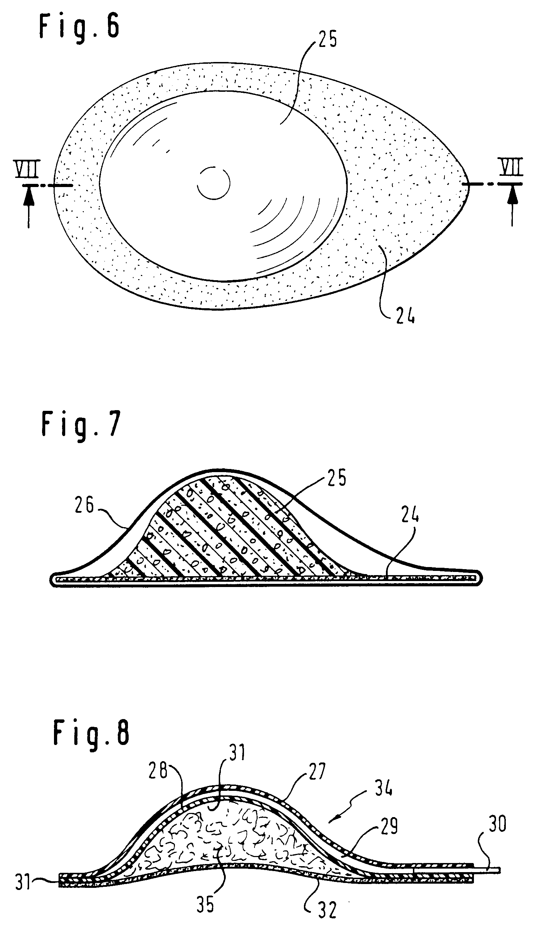

Fig. 6 zeigt in Draufsicht eine Ausführungsform einer sogenannten Leichtprothese, bei welcher auf einer Schmelzklebefolie 24 ein mehr oder weniger starrer zusätzlicher Prothesenkörper 25 aus Schaumstoff oder dergleichen angeordnet ist, der bei Erwärmung der Schmelzklebefolie 24 mit dieser verbunden wird. Der zusätzliche Prothesenkörper 25 kann auch direkt auf der Folie, beispielsweise durch Aufschäumen, erzeugt werden.

Fig. 7 ist der Schnitt VII-VIi nach Fig. 6 und zeigt in dieser Lage die Anordnung des zusätzlichen Prothesenkörpers 25 auf der Schmelzklebefolie 24. Die gesamte Prothese kann - wie andere bekannte Prothesen auch - von einer Hülle 26 umschlossen sein.

Fig. 8 ist ein Schnitt ähnlich Fig. 7 bei einer abgewandelten Ausführungsform. Bei dieser Prothese wird ein schalenförmiger aus zwei Polyurethanfolien 27 und 28 bestehender Prothesenkörper 34 verwendet, wobei zwischen den beiden Polyurethanfolien 27 und 28 eine ebenfalls schalenförmige Kammer 29 gebildet wird, in welche zum Zwecke der Aussteifung über ein Zuführrohr 30 Silikon oder dergleichen eingebracht werden kann.

Aufgrund der Schalenform weist der aus den Polyurethanfolien 27 und 28 bestehende Prothesenkörper 34 eine vorzugsweise dem Körper der Trägerin zugewandte Höhlung 31 auf, die mit Schaumstoff, Watte oder dergleichen ausgesteift sein kann. Die Höhlung 31 kann dann wieder durch eine Schmelzklebefolie 32 abgedeckt sein, die in der beschriebenen Weise eine Verbindung im Randbereich 33 mit dem schalenförmigen Prothesenkörper 34 eingeht. Die beiden Folien 27 und 28 des schalenförmigen Prothesenkörpers 34 können zuvor miteinander verschweißt worden sein.

## Patentansprüche

1. Verfahren zur Herstellung von Brustprothesen, die einen Beutel aus zwei an ihren Rändern miteinander verbundenen Polyurethanfolien aufweisen, der mit Silikon gefüllt ist, dadurch gekennzeichnet, daß zwischen die beiden Polyurethanfolien (4,6) eine Schmelzklebefolie (5) eingelegt wird, die mit einer Ausnehmung (9) versehen ist und die Form eines Ringes hat, wobei die drei so übereinander angeordneten Folien (4,5,6) auf den Rand (8b) eines Hohlkörperwerkzeuges (8) aufgelegt werden, worauf das Werkzeug (8) unter Offenlassen eines Zuführkanales (7) geschlossen wird und anschließend die Füllung erfolgt, indem das Silikonmaterial über den Zuführkanal (7) zwischen die beiden äußeren Folien (4,6) eingeführt wird, worauf während der Temperaturbehandlung des Werkzeuges (8) für den Vernetzungsvorgang des Silikons die Verbindung zwischen den drei Folien (4,5,6) erfolgt.

2. Verfahren nach Anspruch 1, dadurch gekennzeichnet, daß die Schmelzklebefolie (5) ebenfalls aus Polyurethan besteht.

3. Verfahren nach Anspruch 1 oder 2, dadurch gekennzeichnet, daß der Zuführkanal die Form eines Zuführrohres (7) hat.

4. Verfahren nach einem der vorhergehenden Ansprüche, dadurch gekennzeichnet, daß die als Boden- und Deckfolie dienenden äußeren Polyurethanfolien (4,6) eine Stärke bis 0,08 mm haben, während die Schmelzklebefolie etwa 0,03 mm stark ist.

5. Verfahren nach Anspruch 1 zur Herstellung von Brustprothesen, bei denen übereinander mehrere Kammern angeordnet sind, dadurch gekennzeichnet, daß die zwischen den beiden äußeren Polyurethanfolien (4,6) angeordnete Schmelzklebefolie (13) durchgehend ohne Ausnehmung ausgebildet ist und zwei Kammern (14,15) voneinander trennt.

6. Verfahren nach Anspruch 5, dadurch gekennzeichnet, daß die beiden zwischen der Schmelzklebefolie (13) und der Bodenfolie (4) einerseits und der Schmelzklebefolie (13) und der Deckfolie (6) andererseits gebildeten Kammern (14,15) getrennt über zwei voneinander getrennte Zuführkanäle (11,12) gefüllt werden.

7. Verfahren nach Anspruch 6, dadurch gekennzeichnet, daß die Füllungen verschiedener Kammern (14,15) unterschiedliche Härte, Dichte und/oder Konsistenz aufweisen.

8. Verfahren nach einem der Ansprüche 5 bis 7, dadurch gekennzeichnet, daß innerhalb der Brustprothese drei und mehr Kammern (18,19,20) übereinander angeordnet werden, wobei die dritte (19) und die folgenden Kammern jeweils zwischen zwei benachbarten Klebefolien (16,17) gebildet werden.

9. Verfahren nach Anspruch 1, dadurch gekennzeichnet, daß Schmelzklebefolien (24) für die Herstellung von Leichtprothesen verwendet werden.

10. Verfahren nach Anspruch 9, dadurch gekennzeichnet, daß ein zusätzlicher Prothesenkörper (25) aus Schaumstoff oder dergleichen auf die durchgehend oder ringförmig ausgebildete Schmelzklebefolie (24) aufgebracht und mit dieser verbunden wird.

11. Verfahren nach Anspruch 10, dadurch gekennzeichnet, daß der Schaumstoffkörper (25) auf der Schmelzklebefolie (24) beispielsweise durch Aufschäumen erzeugt wird.

12. Verfahren nach Anspruch 10, dadurch gekennzeichnet, daß der zusätzliche Prothesenkörper (25) aus einem leicht verformbaren Material, wie Watte oder dergleichen besteht, und auf der Schmelzklebefolie (24) erzeugt wird.

13. Verfahren nach Anspruch 9 oder 10, dadurch gekennzeichnet, daß ein silikongefüllter schalenförmiger Prothesenkörper (34) verwendet wird, der aus zwei Polyurethanfolien (27,28) besteht und eine Höhlung aufweist, die insbesondere dem Körper der Trägerin zugewandt ist.

14. Verfahren nach Anspruch 13, dadurch gekennzeichnet, daß die Höhlung (31) durch einen Ergänzungskörper aus Schaumstoff oder dergleichen oder durch Watte oder durch einen aus einer Kombination solcher Materialien bestehenden Ergänzungskörper ausgefüllt und/oder ausgesteift ist.

15. Verfahren nach Anspruch 14, dadurch gekennzeichnet, daß die Verbindung zwischen dem schalenförmigen Prothesenkörper (34) durch eine Schmelzklebefolie (32) herbeigeführt wird, welche den Ergänzungskörper (35) trägt.

16. Verfahren nach einem der Ansprüche 9 bis 15, dadurch gekennzeichnet, daß die Verbindung zwischen der Schmelzklebefolie (24) und dem zusätzlichen Prothesenkörper (25) und/oder dem Ergänzungskörper (35) und/oder dem schalenförmigen Prothesenkörper (34) durch Erhitzen in einem entsprechenden Werkzeug hergestellt wird.

## Claims

1. Manufacturing process for breast prostheses comprising a bag of two polyurethane foils, which are joined at their edges and filled with silicone, **characterized in that** a thermoplastic adhesive foil (5) is inserted between the two polyurethane foils (4, 6), which is provided with a cutout (9) and ringshaped, and that the three foils (4, 5, 6) thus stacked are placed on the edge (8b) of a cavity tool (8), whereafter the tool (8) is closed whilst leaving open a feed channel (7), and thereafter the filling is carried out in that the silicone material is fed via the feed channel (7) between the two outer foils (4, 6), whereafter the three foils (4, 5, 6) are joined during heat treatment of the tool (8) for the purpose of bonding the silicone.

2. Process according to claim 1, **characterized in that** the thermoplastic adhesive foil (5) is also made of polyurethane.

3. Process according to claim 1 or 2, **characterized in that** the feed channel is shaped like a feed pipe (7).

4. Process according to one of the above claims, **characterized in that** the outer polyurethane foils (4, 6) serving as base and cover foils have a thickness of up to 0.08 mm, whilst the thermoplastic adhesive foil is approximately 0.03 mm thick.

5. Process according to claim 1 for the manufacture of breast prostheses, having a plurality of chambers arranged on top of each other, **characterized in that** the thermoplastic adhesive foil (13), which is arranged between the two outer polyurethane foils (4, 6), is arranged continuously without cutout and separates two chambers (14, 15) from each other.

6. Process according to claim 5, **characterized in that** the two chambers which have been formed on the one hand between the thermoplastic adhesive foil (13) and the bottom foil (4) and on the other hand between the thermoplastic adhesive foil (13) and the top foil (6) are filled separately via two feed channels (11, 12) which are separated from each other.

7. Process according to claim 6, **characterized in that** the fillings of different chambers (14, 15) are of different hardness, density and/or consistency.

8. Process according to one of claims 5 to 7, **characterized in that** three or more chambers (18, 19, 20) are stacked inside the breast prosthesis and that the third (19) and following chambers are established between two adjacent adhesive foils (16, 17).

9. Process according to claim 1, **characterized in that** thermoplastic adhesive foils (24) are used for the manufacture of lightweight prostheses.

10. Process according to claim 9, **characterized in that** an additional prosthesis body (25) of foam material or the like is added to the continuous or ringshaped thermoplastic adhesive foil (24) and attached thereto.

11. Process according to claim 10, **characterized in that** the foam material body (25) is produced on the thermoplastic adhesive foil (24), for example by foaming.

12. Process according to claim 10, **characterized in that** the additional prosthesis body (25) is made of an easily deformable material, such as cotton wool or the like, and produced on the thermoplastic adhesive foil (24).

13. Process according to claim 9 or 10, **characterized in that** a silicone filled cupshaped prosthesis body (34) is used, which is composed of two polyurethane foils (27, 28) and comprises a cavity which is in particular oriented towards the body of the wearer.

14. Process according to claim 13, **characterized in that** the cavity (31) is filled and/or reinforced by a supplementary body of foam material or the like or by cotton wool or by a supplementary body composed of a combination of such materials.

15. Process according to claim 14, **characterized in that** the connection between the cupshaped prosthesis body (34) is achieved by a thermoplastic adhesive foil (32) which supports the supplementary body (35).

16. Process according to one of claims 9 to 15, **characterized in that** the connection between the thermoplastic adhesive foil (24) and the additional prosthesis body (25) and/or the supplementary body (35) and/or the cupshaped prosthesis body (34) is achieved by heating in a suitable tool.

## Revendications

1. Procédé de fabrication de prothèses mammaires, qui comportent un sac fait de deux feuilles de polyuréthanne assemblées entre elles sur leurs bords, lequel sac est rempli de silicone, caractérisé en ce qu'entre les feuilles de polyuréthanne (4, 6) est placée une feuille adhésive fusible (5) qui est pourvue d'un évidement (9) et qui a la forme d'un anneau, les trois feuilles (4, 5, 6) ainsi superposées étant placées sur la bord (8b) d'un outil à corps creux (8), l'outil (8) étant fermé en laissant ouvert un canal d'arrivée (7), puis il est procédé au remplissage, par introduction de la matière de silicone par le canal d'arrivée (7) entre les deux feuilles (4, 6) extérieures, après quoi, pendant le traitement thermique de l'outil (8) pour la réticulation du silicone, l'assemblage est réalisé entre les trois feuilles (4, 5, 6).

2. Procédé selon la revendication 1, caractérisé en ce que la feuille adhésive fusible (5) est également en polyuréthanne.

3. Procédé selon la revendication 1 ou 2, caractérisé en ce que le canal d'arrivée à la forme d'un tube d'alimentation (7) .

4. Procédé selon l'une des revendications précédentes, caractérisé en ce que les feuilles de polyuréthanne (4, 6) extérieures, servant de feuille de fond et de feuille de couverture ont une épaisseur pouvant aller jusqu'à 0,08 mm, tandis que la feuille adhésive fusible a une épaisseur d'environ 0 03 mm.

5. Procédé selon la revendication 1 de fabrication de prothèses mammaires dans lesquelles plusieurs chambres sont superposées, caractérisé en ce que la feuille adhésive fusible (13), placée entre les deux feuilles de polyuréthanne (4, 6) extérieures, est réalisée d'un seul tenant sans évidement, et sépare l'une de l'autre deux chambres (14, 15).

6. Procédé selon la revendication 5, caractérisé en ce que les deux chambres (14, 15) formées entre la feuille adhésive fusible (13) et la feuille de fond (4) d'une part et entre la feuille adhésive fusible (13) et la feuille de couverture (6) d'autre part, sont remplies séparément par deux canaux d'alimentation (11, 12) séparés l'un de l'autre.

7. Procédé selon la revendication 6, caractérisé en ce que les garnissages des différentes chambres (14, 15) présentent une dureté une densité et/ou une consistance différentes.

8. Procédé sel on l'une des revendications 5 à 7, caractérisé en ce qu'à l'intérieur de la prothèse mammaire sont superposées trois chambres (18, 19 et plus), la troisième (19) et les chambres suivantes étant formées chacune entre deux feuilles adhésives (16, 17) voisines.

9. Procédé selon la revendication 1, caractérisé en ce qu'on utilise des feuilles adhésives fusibles (24) pour la fabrication de prothèses légères.

10. Procédé selon la revendication 9, caractérisé en ce qu'un corps de prothèse (25) supplémentaire en mousse ou similaire est appliqué sur la feuille adhésive fusible (24) continue ou annulaire et est assemblée avec celle-ci.

11. Procédé selon la revendication 10, caractérisé en ce que le corps en mousse (25) est produit sur la feuille adhésive fusible (24), par exemple par expansion.

12. Procédé selon la revendication 10, caractérisé en ce que le corps de prothèse (25) supplémentaire est fait d'un matériau facilement déformable, tel que de l'ouate ou similaire, et est produit sur la feuille adhésive fusible (24).

13. Procédé selon la revendication 9 ou 10, caractérisé en ce qu'on utilise un corps de prothèse (34) en forme de coquille, rempli de silicone, qui est constitué de deux feuilles de polyuréthanne (27, 28) et présente une cavité qui est tournée en particulier vers le corps de l'utilisatrice.

14. Procédé selon la revendication 13, caractérisé en ce que la cavité (31) est remplie et/ou rendue rigide par un corps de complément en mousse ou similaire ou par de l'ouate ou par un corps de complément constitué d'une combinaison de ces matériaux.

15. Procédé selon la revendication 14, caractérisé en ce que l'assemblage entre le corps de prothèse (34) en forme de coquille est obtenu au moyen d'une feuille adhésive fusible (32), qui porte le corps de complément (35).

16. Procédé selon l'une des revendications 9 à 15, caractérisé en ce que l'assemblage entre la feuille adhésive fusible (24) et le corps de prothèse (25) supplémentaire et/ou le corps de complément (35) et/ou le corps de prothèse (34) en forme de coquille est réalisé par chauffage dans un outil approprié.
